(19) Europäisches Patentamt

European Patent Office

Office européen des brevets

(11) **EP 1 101 108 B1**

(12) **EUROPÄISCHE PATENTSCHRIFT**

(45) Veröffentlichungstag und Bekanntmachung des Hinweises auf die Patenterteilung:
**22.01.2003   Patentblatt 2003/04**

(21) Anmeldenummer: **99931214.3**

(22) Anmeldetag: **30.06.1999**

(51) Int Cl.$^7$: **G01N 33/32**, G01J 3/46

(86) Internationale Anmeldenummer:
**PCT/EP99/04496**

(87) Internationale Veröffentlichungsnummer:
**WO 00/007009 (10.02.2000 Gazette 2000/06)**

(54) **VERFAHREN ZUR ERMITTLUNG DER FARBTONSTABILITÄT VON LACKEN**

METHOD FOR DETERMINING THE SHADE STABILITY OF LACQUERS

PROCEDE POUR DETERMINER LA STABILITE DE NUANCE DE VERNIS

(84) Benannte Vertragsstaaten:
**DE ES FR**

(30) Priorität: **28.07.1998   DE 19833859**

(43) Veröffentlichungstag der Anmeldung:
**23.05.2001   Patentblatt 2001/21**

(73) Patentinhaber: **BASF Coatings Aktiengesellschaft
48165 Münster (DE)**

(72) Erfinder:
• **BIALLAS, Bernd
D-48324 Albesloh (DE)**
• **DUSCHEK, Wolfgang
D-48165 Münster (DE)**
• **ROTZ, Werner
D-48153 Münster (DE)**
• **BERLIN, Harald
D-48301 Nottuln (DE)**

(74) Vertreter: **Fitzner, Uwe, Dr.
Dres. Fitzner & Münch
Rechts- und Patentanwälte
Lintorfer Strasse 10
40878 Ratingen (DE)**

(56) Entgegenhaltungen:
WO-A-98/14778          DE-A- 19 754 547
DE-C- 19 605 520       US-A- 5 583 642

**Beschreibung**

Die vorliegende Erfindung betrifft ein Verfahren zur Ermittlung der Farbtonstabilität von Lacken und zur Optimierung einer Lackformulierung in bezug auf die Farbtonstabilität.

Bei der Verwendung von Farben und Anstrichmitteln für die industrielle Lackierung ist es wichtig, daß ein einmal vorgegebener oder ein vom Kunden gewünschter Farbton möglichst genau und gleichmäßig eingehalten wird, da dem menschlichen Auge auch kleinste Farbabweichungen auffallen. Insbesondere muß darauf geachtet werden, daß Farbabweichungen, die sich aufgrund unterschiedlicher Schichtdicken der Lackierung ergeben, nicht eine intolerable Größe annehmen.

Die Beurteilung einer Farbe beruht dabei im wesentlichen auf der physiologischen Tatsache, daß im menschlichen Auge drei Arten von Rezeptoren mit unterschiedlichen spektralen Empfindlichkeiten vorhanden sind, die grob den drei sog. Grundfarben Rot, Grün und Blau zugeordnet werden können (vgl. Glasurit-Handbuch Lacke und Farben, Vincentz Verlag Hannover 1984, 11. Aufl., Seite 220ff). Um objektive Meßdaten ermitteln zu können, hat man nach statistischen Mittelungen Normspektralwertfunktionen $\underline{x}(\lambda)$, $\underline{y}(\lambda)$ und $\underline{z}(\lambda)$ für die drei Empfindlichkeiten der Rezeptoren festgelegt (CIE-System, DIN 5033). Durch Integration eines beliebigen Farbspektrums $f(\lambda)$ mit diesen Normspektralwertfunktionen kann man die sog. Normfarbwerte $X = \int d\lambda\, \underline{x}(\lambda)\, f(\lambda)$, $Y = \int d\lambda\, \underline{y}(\lambda)\, f(\lambda)$, $Z = \int d\lambda\, \underline{z}(\lambda)\, f(\lambda)$ gewinnen, welche objektive Werte zur Charakterisierung des Farbspektrums f darstellen und als das Ausmaß der Erregung der drei Rezeptortypen im Auge veranschaulicht werden können.

Um das oben beschriebene Farbkoordinatensystem hinsichtlich des Abstandes zweier Farben $X_1$, $Y_1, Z_1$ und $X_2, Y_2, Z_2$ noch besser mit der menschlichen Empfindung in Einklang zu bringen, hat man weiterhin im CIELAB-System (DIN 6174) die Größen L*, a*, b* wie folgt definiert ($X_n, Y_n, Z_n$ sind die Koordinaten des zur Beleuchtung verwendeten Normlichtes):

$$L^* = 116\, (Y/Y_n)^{1/3} - 16$$

$$a^* = 500\, [(X/X_n)^{1/3} - (Y/Y_n)^{1/3}]$$

$$b^* = 200\, [(Y/Y_n)^{1/3} - (Z/Z_n)^{1/3}]$$

Der Gesamtfarbabstand $\Delta E^*$ zweier Farben ist dann im L*-a*-b*-System gegeben durch den euklidischen Abstand

$$\Delta E^* = [(\Delta L^*)^2 + (\Delta a^*)^2 + (\Delta b^*)^2]^{1/2}$$

Wenn in der Praxis das Farbtonverhalten eines Lackes beurteilt werden soll, dann werden nach dem derzeitigen Stand der Technik (z.B. in einem Verfahren nach der DE 196 40 376.6) visuell oder farbmetrisch Probelackierungen vermessen, um z.B. die Werte L*, a*, b* für verschiedene Schichtdicken der Lackierung und verschiedene Beobachtungswinkel zu bestimmen. Man erhält somit eine große und schwer überschaubare Menge an Daten, aus denen abgeschätzt werden muß, ob der untersuchte Lack in seinem Anwendungsbereich eine ausreichende Konstanz des Farbtones aufweist. Insbesondere dürfen in der Praxis zwangsläufig auftretende Schwankungen der Schichtdicke der Lackierung z.B. auf einer Automobilkarosse nicht zu einer auffälligen Veränderung des Farbtones führen. Naturgemäß ist die geschilderte Einschätzung des Lackes anhand der großen Datenmenge aus den Probemessungen nur sehr schwer möglich und erfordert viel Aufwand und Erfahrung in der Auswertung. In jedem Falle ist das Ergebnis stark subjektiv und von den Fähigkeiten des Beurteilers abhängig.

Ein Verfahren zur Ermittlung des Farbtonverhaltens einer Lackschicht in bezug auf die Schichtdicke ist aus dem Dokument DE-C-19605520 zu entnehmen. Hier werden verschiedene farbmetrische Parameter, insbesondere die Helligkeit **L***, einer lackierten Oberfläche in Abhängigkeit von der Schichtdicke gemessen. Die so gewonnenen Meßpunkte werden in ein Korrelationsdiagram zur jeweils zugehörigen Schichtdicke eingetragen. Das Korrelationsdiagram gestattet dann Voraussagen über die visuelle Wirkung der lackierten Oberfläche und daher über die Farbtonstabilität der Lackschicht.

Weiterhin ist die Verwendung des Farbabstands zwischen der Farbe einer Lackschicht und einem Referenzwert zur Bestimmung des Farbtonverhaltens aus dem Dokument US-A-5583642 bekannt.

Die vorliegende Erfindung hat sich daher die Aufgabe gestellt, ein Verfahren zur Ermittlung der Farbtonstabilität von Lacken zu entwickeln, welches ein aussagekräftiges, überschaubares und objektives Maß zur Verfügung stellt, das auch gut mit den Befunden aus einer Lackierlinie korreliert. Des weiteren soll ein

Verfahren zur Optimierung von Lackformulierungen in Hinblick auf die Farbtonstabilität angegeben werden.

Diese Aufgabe wird durch ein Verfahren zur Ermittlung der Farbtonstabilität von Lacken gelöst, bei dem in einem ersten Schritt

a) für verschiedene Schichtdicken SD der Gesamtfarbabstand $\Delta X = \Delta X(SD)$ zwischen der Farbe bei der jeweiligen Schichtdicke SD und der Farbe bei einer vorbestimmten Schichtdicke $SD_0$ gemessen wird.

Demnach werden zunächst die Farbkoordinaten der Lackierung bei einer Referenzschichtdicke $SD_0$ bestimmt. Vorzugsweise wird diese Referenz-

schichtdicke $SD_0$ so gewählt, daß sie am Rand der in der Praxis auftretenden Schichtdickeschwankungen liegt. Wenn z.B. Schwankungen zwischen 10 und 20 µm zu erwarten sind, wäre also $SD_0 = 20$ µm (oder auch noch $SD_0 = 25$ µm) eine geeignete Wahl.

Sodann werden für andere Schichtdicken SD ebenfalls die Farbkoordinaten bestimmt, wobei die Zahl der vermessenen Schichtdicken individuell in Abwägung zwischen dem Aufwand der Messung und der gewünschten Genauigkeit des Ergebnisses festgelegt werden muß. Der Bereich der vermessenen Schichtdicken wird sich im wesentlichen mit dem Intervall der bei der Serienlackierung auftretenden Schichtdickeschwankungen dekken (obiges Beispiel: 10-20 µm).

Mit den Farbkoordinaten zu den Schichtdicken SD kann man dann einen Gesamtfarbabstand $\Delta X(SD)$ zu den Farbkoordinaten der Referenzschichtdicke $SD_0$ bestimmen. Wenn die Farbkoordinaten z.B. Im L\*-a\*-b\*-System ausgedrückt werden, kann $\Delta X$ übereinstimmend mit der bekannten Definition

$$\Delta X = \Delta E^* := [(\Delta L^*)^2 + (\Delta a^*)^2 + (\Delta b^*)^2]^{1/2}$$

gewählt werden. Es sei indes ausdrücklich darauf hingewiesen, daß dies nur eine von mehreren möglichen Wahlen ist. Mit L\*-a\*-b\*-Werten ließen sich z.B. ebenso die Größen

$$\Delta X = \Delta C^* := [a^{*2}+b^{*2}]^{1/2} - [a_0^{*2}+b_0^{*2}]^{1/2}$$

oder

$$\Delta X = \Delta H^* := [(\Delta E^*)^2 - (\Delta L^*)^2 - (\Delta C^*)^2]^{1/2}$$

verwenden.

Im Ergebnis erhält man somit aus Teilschritt a) des erfindungsgemäßen Verfahrens die Schichtdikken-Abhängigkeit des Gesamtfarbabstandes $\Delta X$ (SD) (= Abstand zwischen der Farbe bei der Schichtdicke SD und der Farbe bei der Referenz-Schichtdicke $SD_0$).

Im zweiten Schritt des erfindungsgemäßen Verfahrens werden dann

b) die Steigungen

$$\sigma(SD) = d \, \Delta X / d \, SD$$

als Kenngrößen für die Farbtonstabilität bei der Schichtdicke SD ermittelt.

Die Steigung $\sigma$ (SD) bei der Schichtdicke SD ist demnach die Ableitung des Gesamtfarbabstan-des $\Delta X$ nach der Schichtdicke SD. Diese Steigung gibt an, wie stark die Farbe bei der Schichtdicke SD von der Schichtdickenänderung abhängt.

Überraschenderweise hat sich gezeigt, daß die so ermittelte Kenngröße $\sigma$ (SD) besonders aussagekräftig in bezug auf die Farbtonstabilität des Lakkes ist. Dabei ist von besonderem Vorteil, daß in ihr drei Farbkoordinaten (z.B. L\*, a\*, b\*) zu einem einzigen Wert zusammengefaßt werden. Die Beurteilung der Farbtonstabilität Ist also nicht mehr der Erfahrung oder dem Gefühl eines Auswerters überlassen, sondern kann anhand einer objektiv und reproduzierbar bestimmbaren Kenngröße erfolgen.

Die Durchführung des erfindungsgemäßen Verfahrens kann vorzugsweise so erfolgen, daß die Farbmaßzahlen L\*, a\*, b\* mit einer geeigneten Apparatur unmittelbar gemessen werden und der Gesamtfarbabstand $\Delta X$ hieraus berechnet wird.

Die Messung des Gesamtfarbabstandes $\Delta X$ und/oder der Farbwerte L\*, a\*, b\* kann unter verschiedenen Beobachtungswinkeln zwischen 0° und 180° vorgenommen werden, vorzugsweise unter 15°, 25°, 45°, 75° und 110°. Diese Vorgehensweise ist dann erforderlich, wenn der Farbeindruck eines Lackes von der Beobachtungs- und/oder Beleuchtungsrichtung abhängig ist. Letzteres ist bei vielen Lacken, insbesondere den sog. Effektlackierungen der Fall.

Es hat sich in den Fällen der Winkelabhängigkeit gezeigt, daß aus den $\Delta X_\alpha$ ein (vorzugsweise geometrischer) Mittelwert $\Delta X_{gesamt}$ gebildet werden kann, der ohne Einbußen an der Aussagekraft der Einzelwerte die Farbtonstabilität anzeigt. Anhand dieses Wertes $\Delta X_{gesamt}$ kann die

Steigung $\sigma_{gesamt}(SD)$ bestimmt werden.

Alternativ ist es denkbar, daß die Kenngrößen $\sigma_\alpha(SD)$ bezüglich der Beobachtungswinkel $\alpha$ gemittelt werden. Eine sehr empfindliche Kenngröße $\sigma_{max(\alpha)}(SD)$ ergibt sich weiterhin dann, wenn das Maximum von $\sigma$ über alle Beobachtungswinkel gebildet wird.

Weiterhin ist es möglich, daß die Kenngrößen $\sigma(SD)$ nicht für alle Schichtdicken SD getrennt ausgewertet werden. Auch hier ist es denkbar, die Kenngrößen über das interessierende Schichtdikkeninterval zu mitteln, um so den Mittelwert $<\sigma>$ zu erhalten. Alternativ zur Mittelung könnte indes auch das Maximum $\sigma_{max(SD)}$ von $\sigma$ über alle Schichtdikken SD im Intervall betrachtet werden. Beide Methoden liefern im Ergebnis eine einzige Zahl als Charakteristikum für die Farbtonstabilität des Lakkes.

Diese oben geschilderten Vorteile lassen sich auch dann erzielen, wenn man in Schritt b) statt der Kenngröße $\sigma(SD)$

b') die Grenzschichtdicke $SD_{lim}$, bei der der Gesamtfarbabstand $\Delta X(SD)$ ein vorgegebenes Limit

$\Delta X_0$ unterschreitet, als Kenngrößen für die Farbtonstabilität des Lackes ermittelt.

Bei dieser Methode entfällt von vornherein die Schichtdicken-Abhängigkeit der Kenngröße, und man erhält einen einzigen charakteristischen Zahlenwert $SD_{lim}$. Dieser gibt anschaulich darüber Auskunft, ob im Bereich der praxisüblichen Prozeßschichtdicke ein farbtonstabiles Verhalten vorliegt. Liegt beispielsweise die praxisübliche Prozeßschichtdicke im Bereich von 12 bis 15 μm, dann sollte $SD_{lim} < 12$ μm sein.

Das Limit $\Delta X_0$ wird in der Praxis so gewählt werden, daß innerhalb seiner Grenzen Farbabweichungen auf einem lackierten Gegenstand gerade noch tolerabel wären. Wenn demnach die industrielle Lackierung Schichtdickeschwankungen in einem Intervall $[SD_{min}, SD_{max}]$ erwarten läßt, so wird man z.B. $SD_0 = SD_{max}$ wählen und die Farbtonstabilität des Lackes als hinreichend betrachten, wenn sich in dem erfindungsgemäßen Verfahren ein Wert $SD_{lim} < SD_{min}$ ergibt.

Die Durchführung des zweiten erfindungsgemäßen Verfahrens mit Schritt b') kann vorzugsweise so erfolgen, daß die Farbmaßzahlen L*, a*, b* mit einer geeigneten Apparatur unmittelbar gemessen werden und der Gesamtfarbabstand $\Delta X$ hieraus berechnet wird.

Die Messung des Gesamtfarbabstandes $\Delta X$ und/oder der Farbwerte L*, a*, b* kann unter verschiedenen Beobachtungswinkeln zwischen 0° und 180° vorgenommen werden, vorzugsweise unter 15°, 25°, 45°, 75° und 110°. Diese Vorgehensweise ist dann erforderlich, wenn der Farbeindruck eines Lackes von der Beobachtungs- und/oder Beleuchtungsrichtung abhängig ist. Letzteres ist bei vielen Lacken, insbesondere den sog. Effektlackierungen der Fall.

In den Fällen der Winkelabhängigkeit ist es denkbar, daß die Grenzschichtdicken $SD_{lim,\alpha}$ bezüglich der Beobachtungswinkel $\alpha$ gemittelt werden.

Es hat sich indes gezeigt, daß der höchste (= am nächsten bei $SD_0$ gelegene) Wert $SD_{lim,\alpha}$ als eine die Stabilität charakterisierende Größe angesehen werden kann. D.h., daß sie diejenige Schichtdicke angibt, die im Prozeß überschritten werden muß, um Farbtonstabilität zu gewährleisten.

Die Erfindung betrifft weiterhin ein Verfahren zur Optimierung einer Lackformulierung in bezug auf die Farbtonstabilität in einem Schichtdickenintervall $[SD_{min}, SD_{max}]$.

Bei der Entwicklung neuer Lackformulierungen z.B. für die Serienlackierung von Automobilen ist auf zahlreiche Anforderungen zu achten. Eine davon ist, daß in dem Schichtdickenbereich, in dem die Lackierung In der Praxis aufgetragen wird, keine auffälligen und störenden Schwankungen des von der Schichtdicke abhängigen Farbtons auftreten. Daher muß eine Lackformulierung gegebenenfalls solange verändert (optimiert) werden, bis sie eine ausreichende Farbtonstabilität hat. Eine derartige Optimierung ist mit dem erfindungsgemäßen Verfahren vereinfacht möglich. Bei diesem Verfahren werden

    a) mindestens zwei Varianten der Lackformulierung hergestellt,

    b) von jeder Variante die Kenngrößen $\sigma(SD)$ der Farbtonstabilität im Schichtdickenintervall $[SD_{min}, SD_{max}]$ mit einem oben beschriebenen Verfahren ermittelt, und

    c) diejenige Variante als endgültige Lackformullerung gewählt, welche

    c1) den kleinsten Wert $\sigma(SD)$ auf dem Schichtdickenintervall $[SD_{min}, SD_{max}]$ hat, oder

    c2) den kleinsten Mittelwert $\langle\sigma\rangle$ über das Schichtdickenintervall $[SD_{min}, SD_{max}]$ hat, oder

    c3) den kleinsten Maximalwert $\sigma_{max(SD)}$ auf dem Schichtdickenintervall $[SD_{min}, SD_{max}]$ hat.

Das erfindungsgemäße Optimierungsverfahren erlaubt auf einfache, reproduzierbare und automatisierbare Weise, eine Lackformulierung zu optimieren. Dies gelingt insbesondere dadurch, daß es nur eine einzige Größe $\sigma$ verwendet, die unmittelbar als Optimierungskriterium (Zielgröße oder Kostenfunktion) verwendet werden kann.

Sofern die Kenngröße $\sigma$ wie oben beschrieben sogleich als Schichtdicken-Mittelwert oder als Maximalwert über ein Schichtdickenintervall definiert wurde, vereinfachen sich die Schritte c2) und c3) unmittelbar zur Auswahl der Lackformulierung mit dem minimalen $\sigma$.

Die Variation der Lackformulierungen kann so erfolgen, daß die Kenngröße $\sigma$ nach einem Gradientenverfahren mit Veränderungen der Lackformulierung minimiert wird.

Das erfindungsgemäße Optimierungsverfahren wird vorzugsweise so lange durchgeführt, bis der nach c1), c2) oder c3) herangezogene Wert $\sigma$ kleiner als $0,15$ μm$^{-1}$, vorzugsweise kleiner als $0,1$ μm$^{-1}$, ist, wobei in diesem Falle $\sigma$ speziell über den Gesamtfarbabstand $\Delta E^*$ im L*-a*-b*-System bestimmt wird. Für andere Definitionen von $\sigma$ lassen sich entsprechende Wertvorgaben ermitteln.

Selbstverständlich sind weitere Abwandlungen des erfindungsgemäßen Optimierungsverfahrens denkbar. Insbesondere ist es auch möglich, dieses mit der Grenzschichtdicke $SD_{lim}$ als Optimierungsmaß durchzuführen, d.h. es wird diejenige Lackformulierung gesucht bzw. gewählt, die die besseren Werte von $SD_{lim}$ hat. Abbruchkriterium der Optimierung wäre in diesem Fall das Erreichen eines Lackes, dessen $SD_{lim}$ zur Abdeckung des gesamten interessierenden Schichtdickenbereich führt.

Nachfolgend wird die vorliegende Erfindung mit Hilfe der Figuren beispielhaft erläutert.

Fig. 1 zeigt einen schematisierten Verlauf des Gesamtfarbabstandes $\Delta E^*(SD)$.

Fig.2 zeigt einen gemessenen Verlauf.

In den Figuren ist auf der horizontalen Achse die Schichtdicke SD zwischen den Werten 0 und 25 µm aufgetragen. Für industrielle Beschichtungen ist in etwa der Bereich zwischen 10 und 20 µm interessant.

Auf der vertikalen Achse ist Gesamtfarbabstand $\Delta E(SD)$ aufgetragen, welcher in den Beispielen dem im L*-a*-b*-System definierten Wert

$$\Delta E^* = [(\Delta L^*)^2 + (\Delta a^*)^2 + (\Delta b^*)^2]^{1/2}$$

entspricht. Der Farbabstand ist auf die Farbe bei der Schichtdicke $SD_0$ bezogen, so daß die Farbabstandskurve bei $SD_0$ eine Nullstelle hat.

Ein vorgegebenes Limit $\Delta E_0 = 1$ wird bei der Schichtdicke $SD_{lim}$ überschritten. Oberhalb von $SD_{lim}$ wäre die Beschichtung also noch als ausreichend farbtonstabil zu betrachten.

Weiterhin stellt die Steigung $\sigma$ der (für einen Punkt beispielhaft eingezeichneten) Tangente an die $\Delta E^*$-Kurve eine erfindungsgemäße Kenngröße für die Farbtonstabilität dar. Überschreitet diese Steigung einen vorgegebenen Wert auf dem interessierenden Intervall, so ist der Lack nicht ausreichend farbtonstabil und prozeßsicher verarbeitbar. Es hat sich herausgestellt, daß $\sigma$ kleiner als 0,15 µm$^{-1}$ sein sollte.

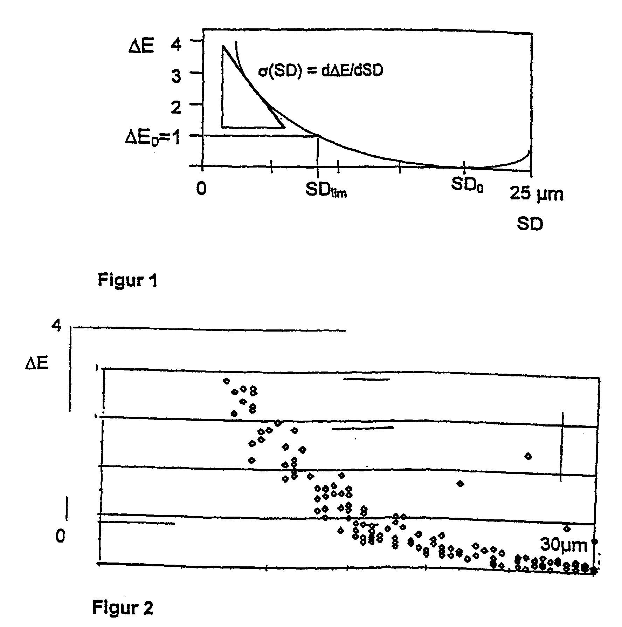

Figur 2 zeigt dieselbe Darstellung mit gemessenen Werten für Basislacke, die in der Automobillackierung verwendet werden. In Hinblick auf eine gute Korrelation der gewonnenen Laborwerte mit den Ergebnissen im realen Einsatz an der Lackierlinie ist dabei darauf zu achten, daß im Meßverfahren der fragliche Basislack auf dem adäquaten Füller appliziert wird.

**Patentansprüche**

1. Verfahren zur Ermittlung der Farbtonstabilität von Lacken enthaltend die Schritte

> a) für verschiedene Schichtdicken SD wird der Gesamtfarbabstand $\Delta X(SD)$ zwischen der Farbe bei der Schichtdicke SD und der Farbe bei einer vorbestimmten Schichtdicke $SD_0$ gemessen,
> b) die Steigungen
>
> $$\sigma (SD) = d\,\Delta X/d\,SD$$
>
> werden als Kenngrößen für die Farbtonstabilität bei der Schichtdicke SD ermittelt.

2. Verfahren nach Anspruch 1, **dadurch gekennzeichnet, daß** die Farbmaßzahlen L*, a*, b* unmittelbar gemessen und hieraus der Gesamtfarbabstand $\Delta X$ berechnet wird.

3. Verfahren nach einem der Ansprüche 1 oder 2, **dadurch gekennzeichnet, daß** die Messung der Gesamtfarbabstände $\Delta X$ und/oder der Farbwerte L*, a*, b* unter verschiedenen Beobachtungswinkeln erfolgt, vorzugsweise unter 15°, 25°, 45°, 75° und 110°.

4. Verfahren nach Anspruch 3, **dadurch gekennzeichnet, daß** die Gesamtfarbabstände $\Delta X_\alpha$ bezüglich der Beobachtungswinkel gemittelt werden und aus der resultierenden Funktion $\Delta X_{gesamt}(SD)$ eine Kenngröße $\sigma_{gesamt}(SD)$ berechnet wird.

5. Verfahren nach einem der Ansprüche 1 bis 4, **dadurch gekennzeichnet, daß** die Kenngrößen $\sigma$ (SD) über ein Schichtdickenintervall zu einem Wert $<\sigma>$ gemittelt werden oder daß auf dem Schichtdickenintervall der maximale Wert $\sigma_{max(SD)}$ von $\sigma(SD)$ bestimmt wird.

6. Verfahren zur Ermittlung der Farbtonstabilität von Lacken enthaltend die Schritte

> a) für verschiedene Schichtdicken SD wird der Gesamtfarbabstand $\Delta X(SD)$ zwischen der Farbe bei der Schichtdicke SD und der Farbe bei einer vorbestimmten Schichtdicke $SD_0$ gemessen,
> b') die Grenzschichtdicke $SD_{lim}$, bei der der Gesamtfarbabstand $\Delta X(SD)$ ein vorgegebenes Limit $\Delta X_0$ unterschreitet, wird als Kenngrößen für die Farbtonstabilität des Lackes ermittelt.

7. Verfahren nach Anspruch 6, **dadurch gekennzeichnet, daß** die Farbmaßzahlen L*, a*, b* unmittelbar gemessen und hieraus der Gesamtfarbabstand $\Delta X$ berechnet wird.

8. Verfahren nach einem der Ansprüche 6 bis 7, **dadurch gekennzeichnet, daß** die Messung der Gesamtfarbabstände $\Delta X$ und/oder der Farbwerte L*, a*, b* unter verschiedenen Beobachtungswinkeln erfolgt, vorzugsweise unter 15°, 25°, 45°, 75° und 110°.

9. Verfahren nach Anspruch 8, **dadurch gekennzeichnet, daß** der Maximalwert der Grenzschichtdicken $SD_{lim,\alpha}$ als Kenngröße für die Farbtonstabilität bestimmt wird.

10. Verfahren zur Optimierung einer Lackformulierung in bezug auf die Farbtonstabilität in einem Schichtdickenintervall, **dadurch gekennzeichnet, daß**

a) mindestens zwei Varianten der Lackformulierung hergestellt werden,

b) von jeder Variante die Kenngrößen $\sigma(SD)$ der Farbtonstabilität im Schichtdickenintervall mit einem Verfahren nach einem der Ansprüche 1 bis 5 ermittelt werden, und

c) diejenige Variante als endgültige Lackformulierung gewählt wird, welche

c1) den kleinsten Wert $\sigma$ auf dem Schichtdickenintervall hat, oder

c2) den kleinsten Mittelwert $<\sigma>$ über das Schichtdickenintervall hat, oder

c3) den kleinsten Maximalwert $\sigma_{max(SD)}$ auf dem Schichtdickenintervall hat.

11. Verfahren nach Anspruch 10,
**dadurch gekennzeichnet, daß** die Optimierung so lange
durchgeführt wird, bis der kleinsten Wert $\sigma$ auf dem Schichtdickenintervall kleiner als 0,15 $\mu m^{-1}$, vorzugsweise kleiner als 0,1 $\mu m^{-1}$, ist, wobei $\sigma$ über den Gesamtfarbabstand $\Delta E^*$ im $L^*$-$a^*$-$b^*$-System bestimmt wird.

**Claims**

1. A method of determining the shade stability of coatings, comprising the steps of

a) measuring, for different film thicknesses FT, the total color difference $\Delta X(FT)$ between the color at the film thickness FT and the color at a predetermined film thickness $FT_0$, and

b) determining the slopes

$$\sigma(FT) = d\,\Delta X/d\,FT$$

as parameters for the shade stability at the film thickness FT.

2. The method as claimed in claim 1, wherein the color coordinates $L^*,a^*,b^*$ are measured directly and from these measurements the total color difference $\Delta X$ is calculated.

3. The method as claimed in either of claims 1 and 2, wherein the total color differences $\Delta X$ and/or the color values $L^*,a^*,b^*$ are measured at different observation angles, preferably at $15°$, $25°$, $45°$, $75°$, and $110°$.

4. The method as claimed in claim 3, wherein the total color differences $\Delta x_\alpha$ are averaged in respect of the observation angles and from the resulting function $\Delta X_{total}(FT)$ a parameter $\sigma_{total}(FT)$ is calculated.

5. The method as claimed in any of claims 1 to 4, wherein the parameters $\sigma(FT)$ are averaged over a film thickness range to give a value $<\sigma>$ or wherein the maximum value $\sigma_{max(FT)}$ of $\sigma(FT)$ is determined in the film thickness range.

6. A method of determining the shade stability of coating materials, comprising the steps of

a) measuring, for different film thicknesses FT, the total color difference $\Delta X(FT)$ between the color at the film thickness FT and the color at a predetermined film thickness $FT_0$, and

b') determining the limiting film thickness $FT_{lim}$ at which the total color difference $\Delta X(FT)$. passes below a preset limit $\Delta X_0$, as parameters for the shade stability of the coating.

7. The method as claimed in claim 6, wherein the color coordinates $L^*,a^*,b^*$ are measured directly and from these measurements the total color difference $\Delta X$ is calculated.

8. The method as claimed in either of claims 6 and 7, wherein the total color differences $\Delta X$ and/or the color values $L^*,a^*,b^*$ are measured at different observation angles, preferably at $15°$, $25°$, $45°$, $75°$, and $110°$..

9. The method as claimed in claim 8, wherein the maximum value of the limiting film thicknesses $FT_{lim,\alpha}$ is determined as a parameter for the shade stability.

10. A method of optimizing a paint formulation in respect of the shade stability in a film thickness range, which comprises

a) preparing at least two variants of the paint formulation,

b) determining for each variant the parameters $\sigma(FT)$ of the shade stability in the film thickness range by a method as claimed in any of claims 1 to 5, and

c) choosing as the final paint formulation the variant which

c1) has the smallest value $\sigma$ in the film thickness range, or

c2) the smallest average $<\sigma>$ over the film thickness range, or

c3) the smallest maximum value $\sigma_{max(FT)}$ in the film thickness range.

**11.** The method as claimed in claim 10, wherein the optimization is implemented until the smallest value σ in the film thickness range is less than 0.15 μm$^{-1}$, preferably less than 0.1 μm$^{-1}$, σ being determined by way of the total color difference ΔE* in the L*-a*-b* system.

**Revendications**

**1.** Procédé pour la détermination de la stabilité de la teinte de peintures, comportant les étapes suivantes

a) on mesure pour diverses épaisseurs de couche SD la distance chromatique totale ΔX(SD) entre la couleur à l'épaisseur de couche respective SD et la couleur à une épaisseur de couche préétablie SD$_0$,
b) on détermine les pentes

$$\sigma(SD) = d\ \Delta X/d\ SD$$

en tant que grandeurs caractéristiques de la stabilité de la teinte à l'épaisseur de couche SD.

**2.** Procédé selon la revendication 1, **caractérisé en ce qu'**on mesure directement les composantes trichromatiques L*, a*, b* et on calcule à partir de celles-ci la distance chromatique totale ΔX.

**3.** Procédé selon la revendication 1 ou 2, **caractérisé en ce que** la mesure des distances chromatiques totales ΔX et/ou des indices chromatiques L*, a*, b* s'effectue sous divers angles d'observation, de préférence sous 15°, 25°, 45°, 75° et 110°.

**4.** Procédé selon la revendication 3, **caractérisé en ce que** les distances chromatiques totales ΔX$_\alpha$ sont déterminées en relation avec les angles d'observation et on calcule une grandeur caractéristique σ$_{totale}$(SD) à partir de la fonction ΔX$_{totale}$(SD) résultante.

**5.** Procédé selon l'une quelconque des revendications 1 à 4, **caractérisé en ce que** les grandeurs caractéristiques σ(SD) sont déterminées sur un intervalle d'épaisseurs de couche pour l'obtention d'une valeur <σ>, ou **en ce qu'**on détermine la valeur maximale σ$_{max(SD)}$ de σ(SD) dans l'intervalle d'épaisseurs de couche.

**6.** Procédé pour la détermination de la stabilité de la teinte de peintures, comportant les étapes suivantes

a) on mesure pour diverses épaisseurs de couche SD la distance chromatique totale ΔX(SD) entre la couleur à l'épaisseur de couche respective SD et la couleur à une épaisseur de couche préétablie SD$_0$,
b) on détermine l'épaisseur de couche limite SD$_{lim}$ à laquelle la distance chromatique totale ΔX(SD) est inférieure à une limite préétablie ΔX$_0$, en tant que grandeurs caractéristiques de la stabilité de la teinte de la peinture.

**7.** Procédé selon la revendication 6, **caractérisé en qu'**on mesure directement les composantes trichromatiques L*, a*, b* et on calcule à partir de celles-ci la distance chromatique totale ΔX.

**8.** Procédé selon la revendication 6 ou 7, **caractérisé en ce que** la mesure des distances chromatiques totales ΔX et/ou des indices chromatiques L*, a*, b* s'effectue sous divers angles d'observation, de préférence sous 15°, 25°, 45°, 75° et 110°.

**9.** Procédé selon la revendication 8, **caractérisé en ce qu'**on détermine la valeur maximale des épaisseurs de couche limites SD$_{lim,\alpha}$ en tant que grandeur caractéristique de la stabilité de la teinte.

**10.** Procédé pour l'optimisation d'une formule de peinture eu égard à la stabilité de la teinte dans un intervalle d'épaisseurs de couche, **caractérisé en ce que**

a) on prépare au moins deux variantes de la formule de peinture,
b) on détermine pour chaque variante les grandeurs caractéristiques σ(SD) de la stabilité de la teinte dans l'intervalle d'épaisseurs de couche par un procédé selon l'une quelconque des revendications 1 à 5, et
c) on choisit comme formule de peinture finale la variante qui

c1) a la plus faible valeur σ dans l'intervalle d'épaisseurs de couche ou
c2) a la plus faible moyenne <σ> dans l'intervalle d'épaisseurs de couche ou
c3) a la plus faible valeur maximale σ$_{max(SD)}$ dans l'intervalle d'épaisseurs de couche.

**11.** Procédé selon la revendication 10, **caractérisé en ce qu'**on effectue l'optimisation jusqu'à ce que la plus faible valeur σ dans l'intervalle d'épaisseurs de couche soit inférieure à 0,15 μm$^{-1}$, de préférence inférieure à 0,1 μm$^{-1}$, σ étant déterminée sur la distance chromatique totale ΔE* dans le système L*-a*-b*.

Figur 1

Figur 2